**Europäisches Patentamt**

**European Patent Office**

**Office européen des brevets**

(19)

(11) **EP 1 481 707 A1**

(12) **EUROPEAN PATENT APPLICATION**

(43) Date of publication:
**01.12.2004 Bulletin 2004/49**

(51) Int Cl.⁷: **A61N 1/368**, A61N 1/362

(21) Application number: **04090210.8**

(22) Date of filing: **27.05.2004**

(84) Designated Contracting States:
**AT BE BG CH CY CZ DE DK EE ES FI FR GB GR
HU IE IT LI LU MC NL PL PT RO SE SI SK TR**
Designated Extension States:
**AL HR LT LV MK**

(30) Priority: **30.05.2003 US 474798**

(71) Applicant: **Biotronik GmbH & Co. KG
12359 Berlin (DE)**

(72) Inventor: **Scharf, Christoph
8627 Grüningen (CH)**

(74) Representative: **Eisenführ, Speiser & Partner
Patentanwälte Rechtsanwälte
Spreepalais am Dom
Anna-Louisa-Karsch-Strasse 2
10178 Berlin (DE)**

(54) **Electrotherapy device**

(57) An implantable device, such as a pacemaker, delivers ventricular pacing based on sensed or paced atrial events. The ventricular pacing occurs after an AV delay, triggered by the atrial event. The AV delay is set to a baseline for a resting heart rate. As the heart rate increases, the AV delay is prolonged to allow for increased filling of the ventricle. This increases cardiac performance for patients with chronic heart failure.

Figure 7

EP 1 481 707 A1

**Description**

The present invention relates to electrotherapy devices for electro-stimulation of a heart and more specifically to implantable medical devices. Even more specifically, the present invention relates to implantable medical devices that pace a ventricular chamber based on a sensed or paced atrial event.

In a healthy heart, an atrial contraction is followed by a ventricular contraction after a natural conduction or delay time called atrio-ventricular delay or AV-delay or AV-interval. Since a natural atrial contraction an electrocardiogram is reflected by a so-called P-wave and the natural contraction of a ventricle is reflected by a R-wave, the natural AV-interval is also called PR-interval.

Electrotherapy devices like cardiac pacemakers or cardioverter/defibrillators mimic this natural behaviour when pacing the heart in an dual chamber mode (DDD-Mode) and in particular in an atrium synchronous mode, where a sensed atrial event (contraction) triggers an AV-delay. At the end of the AV-delay (time out of AV-delay timer) a ventricular pacing pulse is triggered unless a natural ventricular contraction is sensed during the AV-delay leading to inhibition of the triggering of a ventricular pacing pulse. There are numerous attempts to adjust the AV-delay to a patient's physiological need.

In certain implantable medical devices (IMD's) used for pacing, cardioversion, and/or defibrillation referred to herein collectively as ICD's (the term ICD herein is used for pacemakers as well, although in a narrower sense of the word ICD means implantable cardioverter/defibrillator), the device includes a programmer to monitor and rely on certain timers and intervals in order to optimize usage. For example, in various uni-ventricular and bi-ventricular pacing systems, the atrioventricular delay (AV delay) is relied on to determine specific pacing parameters, namely the AV-delay, also called AV-interval. This is true regardless of whether the atrial event triggering the AV interval is paced or sensed. AV delay is the time between an atrial sensed or paced event and the earliest ventricular paced event.

In general, the IMD's are programmed with an AV delay that is in the range of intrinsic conduction. Shortening the AV delay can provide capture of the ventricle, lengthening the delay will allow intrinsic conduction and avoid ventricular pacing, in those conditions (e.g., intermittent heart block) where it is appropriate.

In patients with heart failure and asynchrony in ventricular contraction patterns mostly due to delay in interventricular conduction of electric impulses, pacing has shown to be beneficial. Simultaneous activation of the heart from the intrinsic conduction system and/or electrodes in the right and / or left ventricle provide a resynchronized contraction pattern, thus improving cardiac performance. In the patients the AV delay has to be shorter than intrinsic conduction to ensure ventricular pacing. In order the achieve the maximal benefit for heart failure patients the AV delay is optimized to a baseline for the patient at rest. That is, that patient is monitored when at a resting heart rate and the AV delay is set to optimize hemodynamics. For example, the AV delay is adjusted while the heart is monitored using echocardiography, or another appropriate technique. In most every case, an AV delay shorter than intrinsic conduction is optima1 when considering parameters such as flow velocity and minimization of mitral regurgitation.

In some ICD's the programmed baseline AV delay, once optimized, is static and remains constant regardless of heart rate or patient activity. Conversely, in rate responsive ICD's, the AV delay is adjusted from the baseline as the heart rate is increased. An increase in heart rate occurs for many reasons. For example, heart rate may increase (along with changes in other cardiac responses) due to stress, exertion or exercise. As patients with heart failure and significantly dilated heart chambers often lack a contractility reserve, the primary response to exercise or exertion becomes an increase in heart rate. The dynamic AV delay in conventional pacemakers and ICD's shortens as a response to increased heart rate similarly to the normal physiologic decrease in the PR interval that occurs in a normal heart as the atrial rate increases. Thus, the AV interval decreases linearly from the baseline to a minimum delay, with the linear decrease corresponding to the increase in heart rate.

It is an object of the invention to provide for an electrotherapy device and in particular for an implantable medical device which is capable to deliver a more physiological electrotherapy.

In general this object is achieved by an electrotherapy device like an IMD and specifically a pacemaker or an ICD, which is adapted to increase the relative duration of the AV-interval (AV delay) with respect the total duration of a heart cycle (heart interval, pacing interval), when the heart rate or the pacing increase or is increased, respectively. Therefore, the device of the invention reflects a new paradigm in IMD-design, because currently IMD's do not have the ability to prolong the relative AV delay duration as a response to (an increased) heart rate or adrenergic stimulation.

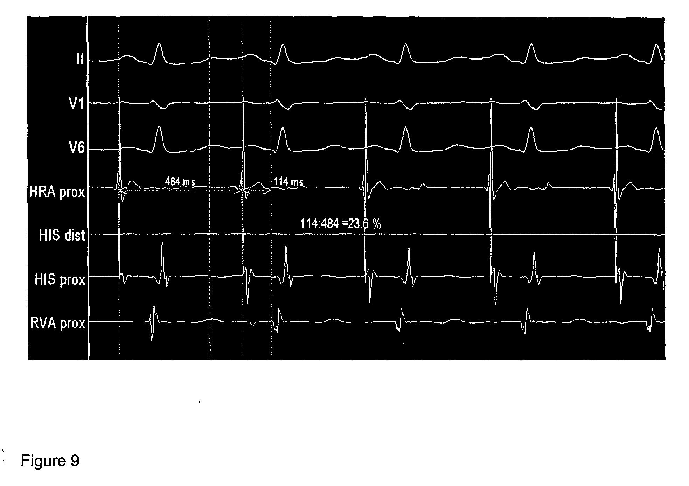

In current dual chamber pacing devices the delays between the pacing stimulus in upper and lower heart chamber is shortened in a linear way as heart rate increases. For instance the dynamic AV delay can be set as a decrease of 1-3 ms per beat increase in heart rate. This leads to the linear curve depicted in Figure 9.

More specifically, the object is achieved by a method for optimizing cardiac performance in a patient having heart failure and an implantable medical device, the method comprising the steps of:

monitoring a heart rate; and

increasing an AV delay from a baseline as the monitored heart rate increases from a resting heart rate.

Alternatively or additionally, the object according to he invention is achieved by a method for optimizing cardiac performance in a patient having an implantable medical device, the method comprising the steps of:

monitoring an indicator of exercise, adrenergic stimulation, or stress;

correlating the monitored indicator to an appropriate heart rate; and

increasing an AV delay from a baseline as the appropriate heart rate increases from a resting heart rate.

In a preferred embodiment, the method additionally comprises the step of monitoring the indicator includes receiving input from one of an activity sensor, minute ventilation sensors, or cardiac impedance sensors.

In a further preferred embodiment, the method additionally comprises the step of pacing the heart at the appropriate heart rate. The method is technically realized by an IMD incorporating means for rate adaption as generally known to the man skilled in the art.

The object is also achieved by an implantable medical device comprising:

sensing means adapted for monitoring a heart rate; and

setting means connected to the sensing means and being adapted for increasing an AV delay from a baseline delay in response to the sensing means as the sensing means indicate an increase in heart rate.

The sensing means preferably form a heart rate monitor.

As a setting means for increasing an AV delay from a baseline delay as the sensing means indicate an increase in heart rate preferably a microprocessor programmed to deliver ventricular pacing after a programmable AV delay is provided. The microprocessor is adapted to generate the programmable AV delay, which includes a baseline correlated to a resting heart rate and a prolonged AV delay correlated to an elevated heart rate.

Preferably the sensing means are adapted to monitor heart rate levels, physical activity levels, stress levels, or adrenergic stimulation levels and are connected to the setting means which are adapted to increase an AV delay from a baseline delay as the sensing means indicates an increase in at least one of the monitored levels.

The implantable medical device preferably further comprises pacing means to pace the heart at an appropriate rate based on the monitored levels.

The invention can be summed up as follows:

The AV Interval of cardiac pacemakers is determined by a percentage of the cycle length at each heart rate. The percentage increases with increasing heart rates. This leads to a nonlinear curve, which corresponds to normal physiology (Figure 10 purple line) . An appropriate algorithm would be:

Set AV Interval = measured Cycle length X percentage X factor

whereas the factor represents an dynamic variable which is rising as a function of decreasing cycle length. The invention shall include:

- Any device incorporating an algorithm which is setting the dynamic AV delay as a relative increase in percentage of cycle length as a response to increase in heart rate.

- Any device incorporating an algorithm incorporating the a relative increase in percentage of cycle length as a response to increase in heart rate to instrinsic heart rate.

- Any device incorporating an algorithm incorporating the a relative increase in percentage of cycle length as a response to increase in heart rate to sensor calculated heart rate.

This algorithm is applicable to any cardiac pacing device (pacemaker or intracardiac defibrillator) which is stimulating the heart in atrium and ventricle.

By such electrotherapy device according to the invention, a more physiologic adaptation of the AV delay to the pacing rate is achieved.

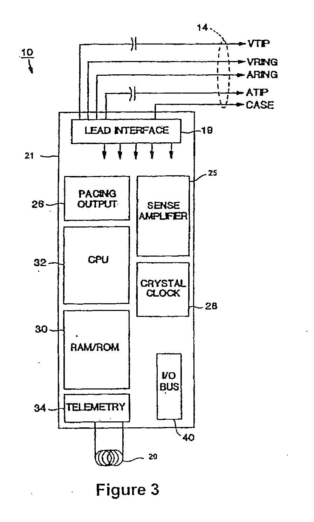

Other than in pacemaker devices the AV node does not shorten linearly in response to an increase in heart rate by adrenergic stimulus in normal patients. Instead of having a constant relation to the cycle length it exhibits an relative prolongation in respect to the cycle length. This means for example that at a heart rate of 774 ms CL, around 77/min the interval from the atrial signal to the onset of ventricular activation (QRS onset) is measured at 144 ms corresponding to 18.6% (Figure 10). When under adrenergic stimulation with isprenalin infusion (B1 agonist) the heart rate increases to 484 ms (124 / min) the interval from the atrial signal to the being of ventricular activation is 114 ms (Figure 3). Therefore, despite the absolute shortening of AV Interval the percentage in respect to the cycle length prolongs.

The invention shall now be described on an exemplary embodiment with reference to the drawings. The method and the device is shown in the figures as follows:

FIG. 1    is an illustration of a body-implantable device system in accordance with the present invention, including a hermetically sealed device implanted in a patient and an external programming unit.

FIG. 2    is a perspective view of the external programming unit of FIG. 1.

FIG. 3    is a block diagram of the implanted device from FIG. 1.

FIG. 4    is a flowchart illustrating the process of implementing a prolonged AV delay.

FIG. 5    is a flowchart illustrating the process of determining the prolonged AV delay, based on heart rate.

FIG. 6    is a flowchart illustrating the process of an implantable device testing and determining an optimal prolonged AV delay.

FIG.7    is representation of AV-delay adaptation according to the prior art versus the AV-delay adaptation according to the invention;

FIG. 8    is representation of ECG-traces representing intracardiac intervals at baseline

FIG. 9    is representation of ECG-traces representing intracardiac intervals with increased heart rate.

## DETAILED DESCRIPTION

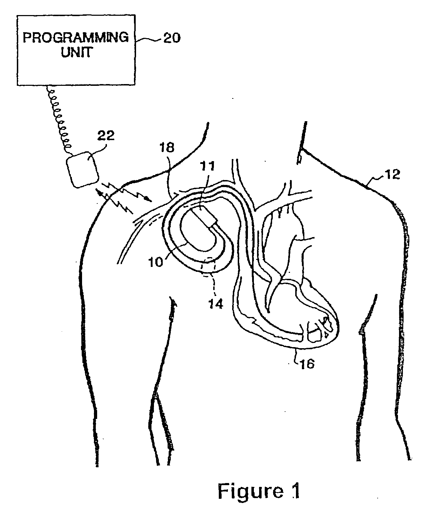

FIG. 1 is an illustration of an implantable medical device system adapted for use in accordance with the present invention. The medical device system shown in FIG. 1 includes implantable device 10, such as a pacemaker, cardioverter and/or defibrillator that has been implanted in patient 12. In accordance with conventional practice in the art, implantable device 10 is housed within a hermetically sealed, biologically inert outer casing, which may itself be conductive so as to serve as an indifferent electrode in the pacemaker's pacing/sensing circuit. One or more pacing and/or sensing leads, collectively identified with reference numeral 14 in FIG. 1 are electrically coupled to pacemaker 10 in a conventional manner and extend into or around the patient's heart 16 via a vein 18. Disposed generally near the distal end of leads 14 are one or more exposed conductive electrodes for receiving electrical cardiac signals and/ or for delivering electrical pacing stimuli to heart 16. As will be appreciated by those of ordinary skill in the art, several leads 14 may be implanted with their distal end(s) situated in or on the atria and/or ventricles of heart 16.

Although the present invention will be described herein in an embodiment which includes a pacemaker, those of ordinary skill in the art having the benefit of the present disclosure will appreciate that the present invention may be practiced in connection with numerous other types of implantable medical device systems, and indeed in any application in which it is desirable to provide a communication link between two physically separated components.

Also depicted in FIG. 1 is an external programming unit 20 for non-invasive communication with implanted device 10 via uplink and downlink communication channels, to be hereinafter described in further detail. Associated with programming unit 20 is a programming head 22, in accordance with conventional medical device programming systems, for facilitating two-way communication between implanted device 10 and programmer 20. In many known implantable device systems, a programming head such as that depicted in FIG. 1 is positioned on the patient's body over the implant site of the device (usually within 2- to 3-inches of skin contact), such that one or more antennae within the head can send RF signals to, and receive RF signals from, an antenna disposed within the hermetic enclosure of the implanted device or disposed within the connector block of the device, in accordance with common practice in the art.

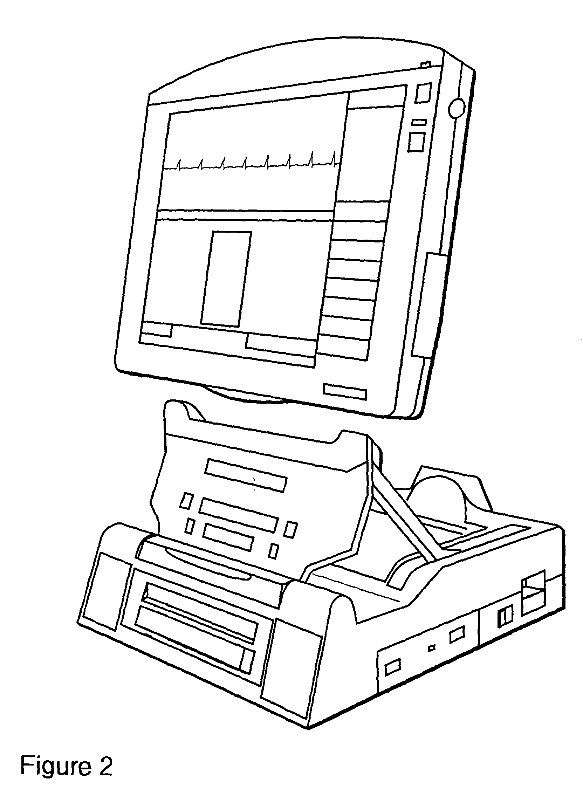

FIG. 2 is a perspective view of programming unit 20 in accordance with the presently disclosed invention. Internally, programmer 20 includes a processing unit (not shown in the Figure) that in accordance with the presently disclosed invention is a personal computer type motherboard, e.g., a computer motherboard including an Intel Pentium 3 microprocessor and related circuitry such as digital memory. The details of design and operation of the programmer's computer system will not be set forth in detail in the present disclosure, as it is believed that such details are well-known to those of ordinary skill in the art.

Referring to FIG. 2, programmer 20 comprises an outer housing 60, which is preferably made of thermal plastic or another suitably rugged yet relatively lightweight material. A carrying handle, designated generally as 62 in FIG. 2, is integrally formed into the front of housing 60. With handle 62, programmer 20 can be carried like a briefcase.

An articulating display screen 64 is disposed on the upper surface of housing 60. Display screen 64 folds down into a closed position (not shown) when programmer 20 is not in use, thereby reducing the size of programmer 20 and protecting the display surface of display 64 during transportation and storage thereof.

A floppy disk drive is disposed within housing 60 and is accessible via a disk insertion slot (not shown). A hard disk drive is also disposed within housing 60, and it is contemplated that a hard disk drive activity indicator, (e.g., an LED, not shown) could be provided to give a visible indication of hard disk activation.

Programmer 20 is equipped with an internal printer (not shown) so that a hard copy of a patient's ECG or of graphics displayed on the programmer's display screen 64 can be generated. Several types of printers, such as the AR-100 printer available from General Scanning Co., are known and commercially available.

In the perspective view of FIG. 2, programmer 20 is shown with articulating display screen 64 having been lifted up into one of a plurality of possible open positions such that the display area thereof is visible to a user situated in front of programmer 20. Articulating display screen is preferably of the LCD or electroluminescent type, characterized by being relatively thin as compared, for example, a cathode ray tube (CRT) or the like.

As would be appreciated by those of ordinary skill in the art, display screen 64 is operatively coupled to the computer circuitry disposed within housing 60 and is adapted to provide a visual display of graphics and/or data under control of the internal computer.

Programmer 20 described herein with reference to FIG. 2 is described in more detail in US. Pat. No. 5,345,362 issued to Thomas J. Winkler, entitled *Portable Computer Apparatus With Articulating Display Panel,* which patent is hereby incorporated herein by reference in its entirety. The Medtronic Model 9790 programmer is the implantable device-programming unit with which the present invention may be advantageously practiced.

FIG. 3 is a block diagram of the electronic circuitry that makes up pulse generator 10 in accordance with the presently disclosed invention. As can be seen from FIG. 3, pacemaker 10 comprises a primary stimulation control circuit 21 for controlling the device's pacing and sensing functions. The circuitry associated with stimulation control circuit 21 may be of conventional design, in accordance, for example, with what is disclosed Pat. No. 5,052,388 issued to Sivula et al., *Method And Apparatus For Implementing Activity Sensing In A Pulse Generator.* To the extent that certain components of pulse generator 10 are conventional in their design and operation, such components will not be described herein in detail, as it is believed that design and implementation of such components would be a matter of routine to those of ordinary skill in the art. For example, stimulation control circuit 21 in FIG. 3 includes sense amplifier circuitry 25, stimulating pulse output circuitry 26, a crystal clock 28, a random-access memory and read-only memory (RAM/ROM) unit 30, and a central processing unit (CPU) 32, all of which are well-known in the art.

Pacemaker 10 also includes internal communication circuit 34 so that it is capable of communicating with external programmer/control unit 20, as described in Fig. 2 in greater detail.

Further referring to FIG. 3, pulse generator 10 is coupled to one or more leads 14 which, when implanted, extend transvenously between the implant site of pulse generator 10 and the patient's heart 16, as previously noted with reference to FIG. 1.

Physically, the connections between leads 14 and the various internal components of pulse generator 10 are facilitated by means of a conventional connector block assembly 11, shown in FIG. 1. Electrically, the coupling of the conductors of leads and internal electrical components of pulse generator 10 may be facilitated by means of a lead interface circuit 19 which functions, in a multiplexer-like manner, to selectively and dynamically establish necessary connections between various conductors in leads 14, including, for example, atrial tip and ring electrode conductors ATIP and ARING and ventricular tip and ring electrode conductors VTIP and VRING, and individual electrical components of pulse generator 10, as would be familiar to those of ordinary skill in the art. For the sake of clarity, the specific connections between leads 14 and the various components of pulse generator 10 are not shown in FIG. 3, although it will be clear to those of ordinary skill in the art that, for example, leads 14 will necessarily be coupled, either directly or indirectly, to sense amplifier circuitry 25 and stimulating pulse output circuit 26, in accordance with common practice, such hat cardiac electrical signals may be conveyed to sensing circuitry 25, and such that stimulating pulses may be delivered to cardiac tissue, via leads 14. Also not shown in FIG. 3 is the protection circuitry commonly included in implanted devices to protect, for example, the sensing circuitry of the device from high voltage stimulating pulses.

As previously noted, stimulation control circuit 21 includes central processing unit 32 which may be an off-the-shelf programmable microprocessor or micro controller, or a custom integrated circuit. Although specific connections between CPU 32 and other components of stimulation control circuit 21 are not shown in FIG. 3, it will be apparent to those of ordinary skill in the art that CPU 32 functions to control the timed operation of stimulating pulse output circuit 26 and sense amplifier circuit 25 under control of programming stored in RAM/ROM unit 30. It is believed that those of ordinary ski11 in the art will be familiar with such an operative arrangement.

With continued reference to FIG. 3, crystal oscillator circuit 28, in the presently preferred embodiment a 32,768-Hz crystal controlled oscillator provides main timing clock signals to stimulation control circuit 21. Again, the lines over which such clocking signals are provided to the various timed components of pulse generator 10 (e.g., microprocessor 32) are omitted from FLG. 3 for the sake of clarity.

It is to be understood that the various components of pulse generator 10 depicted in FIG. 3 are powered by means of a battery (not shown) that is contained within the hermetic enclosure of pacemaker 10, in accordance with common practice in the art. For the sake of clarity in the Figures, the battery and the connections between it and the other components of pulse generator 10 are not shown.

Stimulating pulse output circuit 26, which functions to generate cardiac stimuli under control of signals issued by CPU 32, may be, for example, of the type disclosed in U.S. Pat. No. 4,476,868 to Thompson, entitled *Body Stimulator Output Circuit,* which patent is hereby incorporated by reference herein in its entirety. Again, however, it is believed that those of ordinary skill in the art could select from among many various types of prior art pacing output circuits that would be suitable for the purposes of practicing the present invention.

Sense amplifier circuit 25, which is of conventional design, functions to receive electrical cardiac signals from leads 14 and to process such signals to derive event signals reflecting the occurrence of specific cardiac electrical events, including atrial contractions (P-waves) and ventricular contractions (R-waves). CPU provides these event-indicating signals to CPU 32 for use in controlling the synchronous stimulating operations of pulse generator 10 in accordance with common practice in the art. In addition, these event-indicating signals may be communicated, via uplink transmission, to external programming unit 20 for visual display to a physician or clinician.

Those of ordinary skill in the art will appreciate that pacemaker 10 may include numerous other components and subsystems, for example, activity sensors and associated circuitry. The presence or absence of such additional components in pacemaker 10, however, is not believed to be pertinent to the present invention, which relates primarily to the implementation and operation of communication subsystem 34 in pacemaker 10, and an associated communication subsystem in external unit 20.

In the present embodiment, an AV delay baseline is programmed into the CPU 32. The AV delay baseline is obtained by monitoring a patient at rest and adjusting the delay until cardiac function is optimized. In certain patients, such as those with chronic heart failure, the implantable device 10 will also include a dynamic AV prolongation as a rate responsive function. That is, as heart rate increases, the AV delay is increased; hence, prolonged. For example, in a patient having a resting heart rate of 80 beats per minute (BPM), a typical AV baseline delay may be on the order of 120 milliseconds, which is shorter than the intrinsic rate. As the heart rate increases to 100 BPM, the AV delay may be increased to 140 milliseconds and at 120 BPM the delay may be 180 milliseconds.

In such patients, an increase in the AV delay from baseline due to an increase in heart rate provides significant clinical benefits. In these patients, the filling of the diseased heart is compromised at higher rates (diastole shortening). Thus, the prolonged AV delay affords a greater interval over which filling is allowed to occur. In other words, in such patients, the atrial contraction (A wave) becomes more important. As the AV delay becomes longer the A wave is clearly more prominent and thus improves filling.

This is contrary to current practice, which continues to shorten the AV delay as heart rate increases. As the baseline AV delay is typically on the order of the duration of the average P-wave, further reductions in the AV delay may actually result in nearly simultaneous atrial and ventricular contractions. In those patients with diminished contractile reserve, this serves to further exacerbate the problem. With the present invention, the atrial contraction becomes more important in providing adequate preload as a result of the prolonged AV delay.

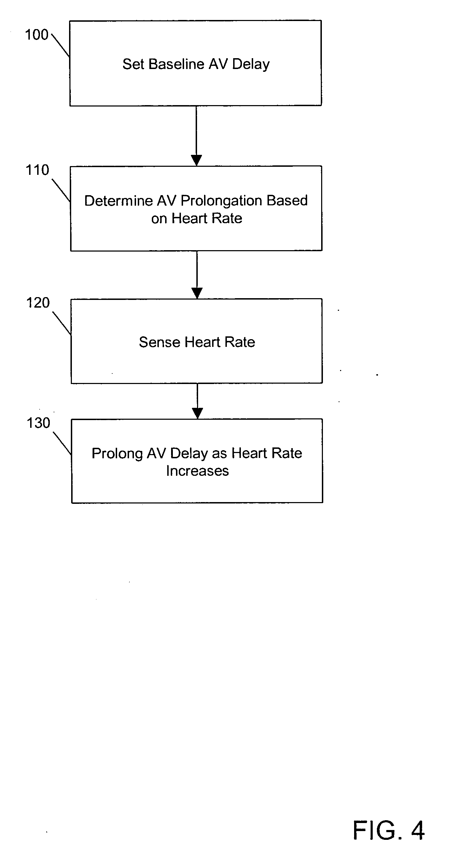

FIG. 4 is a flowchart that illustrates the general process for programming the implanted device 10 to accommodate a prolonged AV delay. At step 100, the baseline AV delay is determined and programmed. The baseline AV delay is determined by monitoring a patient at rest and varying the AV delay. The AV delay producing the most optimal cardiac performance is then selected as the baseline. As previously noted, the implanted device 10 will then utilize this AV delay for that patient whenever the resting heart rate is realized, which will likely be a majority of the time.

Once the baseline AV delay had been determined, the AV prolongation delay is determined 110. The process for making this determination is described in greater detail below. In general, a given prolongation delay is correlated to a specific heart rate or range of heart rates. There may be a linear correlation as heart rate increases or there may be more intermittent correlations. In any event, the AV prolongation delay is preferably determined for a given patient based on observed cardiac characteristics when at that specific heart rate. Of course, with sufficient clinical data, it may be possible to determine guidelines for correlating AV prolongation delays with a general population of patients.

The implanted device 10 will then sense the heart rate 120. If the heart rate is at the resting rate, the baseline AV delay is utilized. However, as the heart rate increases 130, the implanted device 1 will implement the prolonged AV delay, thus increasing cardiac pumping by improving ventricular filling. The heart rate could increase naturally due to exertion or exercise or it could be increased by the programmed pacemaker to account for a sensed increase in activity or the like. Conversely, as the sensed heart rate lowers or returns to the resting rate, the AV delay can be shortened until the base line is reached. There could also continue to be events or conditions where the implanted device 10 will decrease the AV delay from the baseline.

The purpose of prolonging the AV delay is to increase cardiac efficiency by prolonging the ventricular filling period and preload provided by atrial contraction. Generally, the goal will also include maintaining programmed pacing. Thus, if intrinsic conduction occurs (ventricular sensed events) the AV delay will automatically shorten by 10 - 20 msec to ensure ventricular pacing. Alternatively, if relevant a maximum AV delay can be determined to set an upper limit.

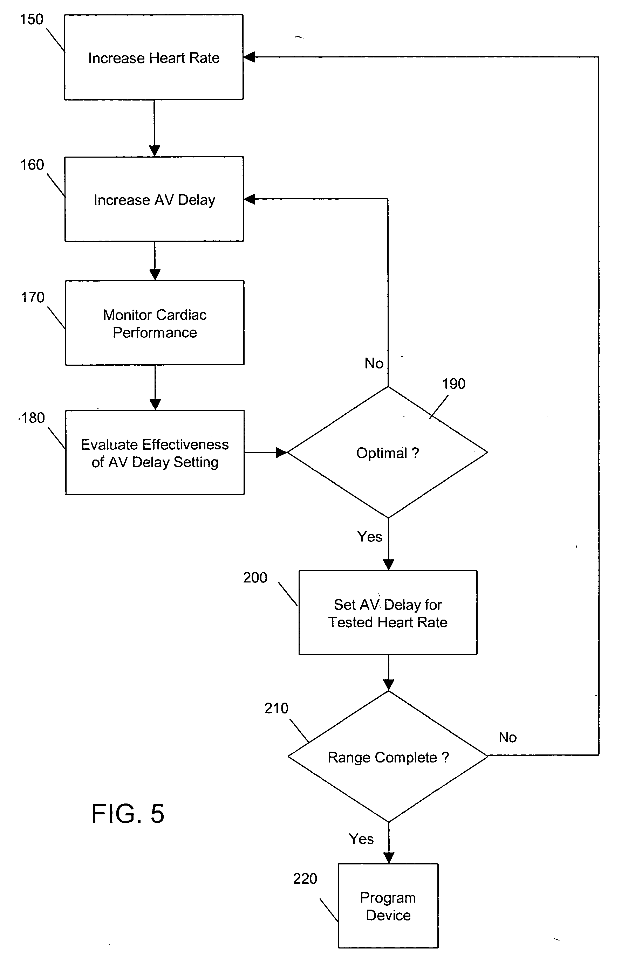

FIG. 5 a flowchart that illustrates the process of determining the prolonged AV delays with respect to heart rate. The implanted device 10 has been implanted and the patient is in a testing environment. The baseline AV delay has been determined and optimized. At 150, the patient's heart rate is increased. For example, the patient could be asked to walk on a tread mill; alternatively, rate increasing drugs could be administered. The AV delay is increased from the baseline by some amount 160. Cardiac performance is monitored 170. For example, an echocardiogram could be obtained. The effectiveness of that particular AV delay setting is then evaluated for that heart rate 180. If after evaluation (which may require sampling various delays and not simply evaluating one in isolation), that AV delay is determined to not be optimal 190, another AV delay is chosen and evaluated 160. Alternatively, a range of delays could all be tried for a given heart rate, the data could then be analyzed and the best delay selected. Once the optima1 delay has been identified 190, that delay is then programmed 200 into the implanted device and is set to correlate to that heart rate.

If that completes the testing 210, the device 10 is programmed and allowed to function 220. If other heart rates need to be evaluated 210, the heart rate is again increased to the next relevant rate 150 and the process is performed again.

As previously mentioned, this process can be performed for any number of different heart rates in a step wise fashion. Alternatively, specific ranges of heart rates can be evaluated. The resultant delays can be specifically identified for each such stepwise heart rate increment; or, linear or other correlations can be extrapolated from a sampling of ranges.

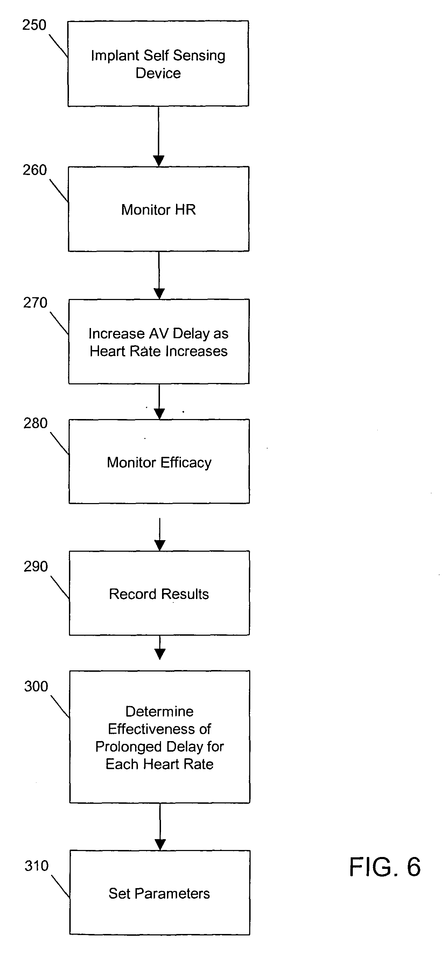

FIG. 6 is a flowchart illustrating the performance a self sensing implanted device 10. That is, the prolonged AV delay could be determined by the implanted - device 10 itself, either once initially, or repeatedly over time to assure optimization.

In use, the device 10 is implanted 250 and monitors the heart rate 260. At this point, the baseline AV delay is already programmed; either through the above described process or by the same process used in this embodiment to set the prolonged AV delay. As the heart rate increases, the device 10 increase the AV delay from the baseline 270 and monitors efficacy 280. These results are recorded 290 and used to determine the optimal setting as other delay data is acquired for a given rate- After a comparison is made, the optimal rate is determined 300 and then programmed 310.

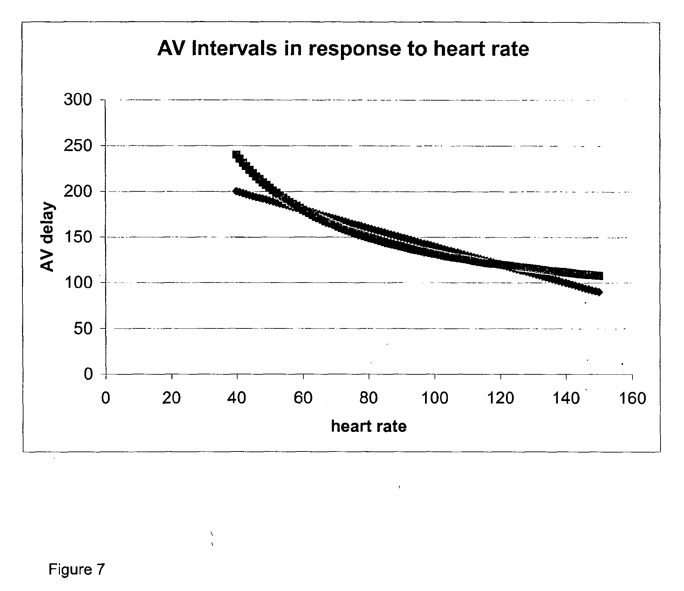

Figure 7 shows the behaviour of current IMDs versus the behaviour of the device according to the invention. The dynamic AV Intervals of current pacemaker devices exhibit a linear shortening in response to rising heart rates (dark line). The Invention comprises any relative increase of AV Interval as percentage of cardiac cycle length. This principle leads to a nonlinear curve (light curve).

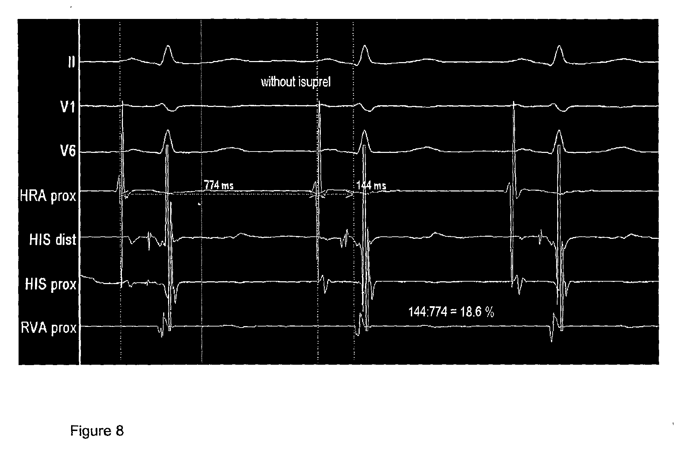

Figure 8 represents ECG-traces (electro cardiograms) showing intracardiac intervals at baseline. The sinus rate is 774 msec. The AV Interval is 144 ms measured from the atrial signal to the beginning of ventricular activation (onset of surface QRS Komplex) which corresponds to 18.6 % of the cycle length. HRA = electrode in high right atrium, HIS dist = electrode on distal His position, His prox = electrode on proximal His position, RVA prox = electrode on right ventricular apex proximal position, II,V1 and V6 = surface ECG leads.

Figure 9 represents ECG-traces (electro cardiograms) showing intracardiac intervals with increased heart rate in a normal person on adrenergic stimulation: The sinus rate is increased to 484 msec upon infusion of B agonist ( Isuprenalin). The AV Interval shortens to 114 msec which corresponds to an increase in relative percentage to 23.6 %. HRA = electrode in high right atrium, HIS dist = electrode on distal His position, His prox = electrode on proximal His position, RVA prox = electrode on right ventricular apex proximal position, II, V1 and V6 = surface ECG leads.

The present invention has been described in the context of monitoring a heart rate and adjusting the AV delay accordingly. Other monitoring or sensing parameters could be evaluated instead of or in addition to the heart rate to determine when to adjust the AV delay. In particular, direct and indirect measures of adrenergic stimulation, exercise, physical activity levels, or stress could be monitored to indicate when the delay should be adjusted. This may be beneficial in patients having sick sinus syndrome or the like where the heart rate may not necessarily correlate as it should to these factors. Such monitoring could, for example, include activity sensors, minute ventilation sensors, cardiac impedance sensors and any other appropriate sensor. An increase in a measure of exercise, stress or adrenergic stimulation, cardiac contractility or ventilation which leads to an increase of paced heart rate in DDDR mode will be accompanied by a gradual increase in AV delay.

Although the present invention has been described with reference to preferred embodiments, persons skilled in the art will recognize that changes may be made in form and detail without departing from the spirit and scope of the invention.

**Claims**

1.  A method for optimizing cardiac performance in a patient having heart failure and an implantable medical device, the method comprising:

    monitoring a heart rate; and

    increasing an AV delay from a baseline as the monitored heart rate increases from a resting heart rate.

2.  An implantable medical device comprising:

    sensing means for monitoring a heart rate; and

    setting means for increasing an AV delay from a baseline delay as the sensing means indicate an increase in heart rate.

3.  The device of claim 2, wherein the increased AV delay facilitates ventricular pacing.

4.  An implantable medical device comprising:

    a heart rate monitor; and

    a microprocessor programmed to deliver ventricular pacing after a programmable AV delay, wherein the programmable AV delay includes a baseline correlated to a resting heart rate and a prolonged AV delay correlated to an elevated heart rate.

5.  A method for optimizing cardiac performance in a patient having an implantable medical device, the method comprising:

    monitoring an indicator of exercise, adrenergic stimulation, or stress;

    correlating the monitored indicator to an appropriate heart rate; and

    increasing an AV delay from a baseline as the appropriate heart rate increases from a resting heart rate.

6.  The method of claim 5, wherein monitoring the indicator includes receiving input from one of an activity sensor, minute ventilation sensors, cardiac impedance sensors.

7.  The method of claim 5, further comprising pacing the heart at the appropriate heart rate.

8.  An implantable medical device comprising:

    sensing means for monitoring heart rate levels, physical activity levels, stress levels, or adrenergic stimulation levels; and

    setting means for increasing an AV delay from a baseline delay as the sensing means indicates an increase in at least one of the monitored levels.

9.  The implantable medical device of claim 8, further comprising pacing means to pace the heart at an appropriate rate based on the monitored levels.

Figure 1

Figure 2

Figure 3

FIG. 4

150 — Increase Heart Rate

160 — Increase AV Delay

170 — Monitor Cardiac Performance

·180 — Evaluate Effectiveness of AV Delay Setting

190 — Optimal ?

No

Yes

200 — Set AV Delay for Tested Heart Rate

210 — Range Complete ?

No

Yes

220 — Program Device

FIG. 5

250 Implant Self Sensing Device

260 Monitor HR

270 Increase AV Delay as Heart Rate Increases

280 Monitor Efficacy

290 Record Results

300 Determine Effectiveness of Prolonged Delay for Each Heart Rate

310 Set Parameters

FIG. 6

AV Intervals in response to heart rate

Figure 7

Figure 8

Figure 9

**European Patent Office**

## PARTIAL EUROPEAN SEARCH REPORT

Application Number

which under Rule 45 of the European Patent Convention EP 04 09 0210
shall be considered, for the purposes of subsequent
proceedings, as the European search report

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | EP 0 607 951 A (VITATRON MEDICAL BV) 27 July 1994 (1994-07-27) * column 6, line 40 - column 7, line 9 * * column 10, line 14 - column 11, line 14; figures 2,6D * | 2-4,8,9 | A61N1/368 A61N1/362 |
| A | US 6 449 510 B1 (ALBERS BERT A ET AL) 10 September 2002 (2002-09-10) * column 11, line 62 - column 14, line 30; figures 7,8 * | 2-4,8,9 | |
| A | MEHTA D. ET AL.: "Optimal atrioventricular delay at rest and during exercise in patients with dual chamber pacemakers: a non-invasive assessment by continuous wave Doppler" BRITISH HEART JOURNAL, vol. 61, 1989, pages 161-166, XP009036744 * page 164, right-hand column, paragraph 2 * * page 165, left-hand column, paragraph 3 * | 2-4,8,9 | |

TECHNICAL FIELDS SEARCHED (Int.Cl.7)

A61N

-/--

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 21 September 2004 | Küster, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

**European Patent
Office**

**INCOMPLETE SEARCH
SHEET C**

**Application Number**

EP 04 09 0210

Claim(s) searched completely:
        2-4,8-9

Claim(s) not searched:
        1,5-7

Reason for the limitation of the search (non-patentable invention(s)):

Article 52 (4) EPC - Method for treatment of the human or animal body by
therapy

**European Patent**
**Office**

**PARTIAL EUROPEAN SEARCH REPORT**

Application Number

EP 04 09 0210

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| A | KOLB H.-J. ET AL.: "Assessment of the optimal atrioventricular delay in patients with dual chamber pacemakers using impedance cardiography and Doppler echocardiography" JOURNAL OF CLINICAL AND BASIC CARDIOLOGY, vol. 2, no. 2, 1999, pages 237-240, XP002297156 * the whole document * ----- | 2-4,8,9 | |
| | | | TECHNICAL FIELDS SEARCHED (Int.Cl.7) |

EPO FORM 1503 03.82 (P04C10)

**ANNEX TO THE EUROPEAN SEARCH REPORT
ON EUROPEAN PATENT APPLICATION NO.**

EP 04 09 0210

This annex lists the patent family members relating to the patent documents cited in the above-mentioned European search report.
The members are as contained in the European Patent Office EDP file on
The European Patent Office is in no way liable for these particulars which are merely given for the purpose of information.

21-09-2004

| Patent document cited in search report | | Publication date | Patent family member(s) | | Publication date |
|---|---|---|---|---|---|
| EP 0607951 | A | 27-07-1994 | US | 5330511 A | 19-07-1994 |
| | | | DE | 69422432 D1 | 10-02-2000 |
| | | | DE | 69422432 T2 | 20-07-2000 |
| | | | EP | 0607951 A2 | 27-07-1994 |
| US 6449510 | B1 | 10-09-2002 | DE | 10119385 A1 | 29-11-2001 |
| | | | FR | 2808211 A1 | 02-11-2001 |

EPO FORM P0459

For more details about this annex : see Official Journal of the European Patent Office, No. 12/82